# EUROPEAN PATENT SPECIFICATION

(11) **EP 1 397 148 B1**
(45) Date of publication and mention of the grant of the patent: **20.09.2006**
(21) Application number: 02721275.2
(22) Date of filing: 27.02.2002
(51) Int. Cl.: A61K 33/24, A61K 31/555, A61K 31/4188, A61P 3/06, A61P 7/00

(54) **CHROMIUM/BIOTIN TREATMENT OF DYSLIPIDEMIA**
CHROMIUM/BIOTIN BEHANDLUNG VON DYSLIPIDEMIA
UTILISATION DE CHROME/BIOTINE POUR LE TRAITEMENT DE L'HYPERGLYCEMIE

(30) Priority: 27.02.2001 US 271881 P
(43) Date of publication of application: 17.03.2004
(73) Proprietor: Nutrition 21, Inc., Purchase, NY 10577-2197 (US)
(72) Inventor: KOMOROWSKI, James R., Trumbull, CT 06611 (US); DE LA HARPE, Jon, New York, Ny 10003 (US); GREENBERG, Danielle, Waccabuc, Ny 10597 (US); JUTURU, Vijaya, Dobbs Ferry, Ny 10522 (US)
(74) Representative: Fennell, Gareth Charles
(86) International application number: PCT/US2002/006877
(87) International publication number: WO 2002/067953

(56) References cited:
- WO-A-02/11564
- WO-A-99/07387
- US-A- 5 597 585
- US-A- 5 789 401
- US-A- 6 140 304
- ANDERSON: "Nutritional Factors Influencing the Glucose/Insulin System: Chromium" JOURNAL OF THE AMERICAN COLLEGE OF NUTRITION, AMERICAN COLLEGE OF NUTRION, WILMINGTON, NC, US, vol. 16, no. 5, 1 October 1997 (1997-10-01), pages 404-410, XP002086777 ISSN: 0731-5724
- MCCARTY M F: "Dietary glycemic index may influence cancer risk by modulating IGF-1 activity: a hypothesis." JOURNAL OF MEDICINAL FOOD 1 (2) 123-140 1998 NUTRIGUARD, 2159 AVENIDA DE LA PLAYA, LA JOLLA, CA 92037, USA. E-MAIL MCCARTY(A)VYREX.COM, 1998, XP001061496

## Description

### Background of the Invention

### Field of the Invention

The present invention relates to the improvement of blood cholesterol and triglyceride levels. More specifically, the invention relates to methods of lowering LDL cholesterol, increasing HDL cholesterol, and decreasing triglyceride levels in the blood by administering doses of a chromium complex and biotin.

### Description of the Related Art

### Dyslipidemia

Dyslipidemias are disorders of lipoprotein metabolism, including lipoprotein overproduction or deficiency. These disorders may be manifested by elevation of the serum total cholesterol, low-density lipoprotein (LDL) cholesterol and triglyceride concentrations, and a decrease in the high-density lipoprotein (HDL) cholesterol concentration. Each year, millions of human beings suffer from the sequelae of hypercholesterolemia. Examples of the afflictions include hypertension, coronary artery disease, congestive heart failure, peripheral vascular disease, aneurysms and death due at least in part to these conditions. Elevated blood cholesterol is one of the major modifiable risk factors for coronary heart disease (CHD), the leading cause of death in the United States. Kannel, W.B. et al. *Declining Cardiovascular Mortality.* Circulation 70:331.338 (1984), CHD is responsible for roughly 480,000 deaths each year, *National Center for Health Statistics.* Annual summary of births, marriages, divorces, and deaths: United States, 1993. Monthly vital statistics report vol 42 no 13. Public Health Service, 1994. Nonfatal myocardial infarction (MI) and angina are similarly a source of substantial morbidity. Secondary physiological effects of hypercholesterolemia include cerebral strokes, compromised liver function, renal artery blockage, senility, male impotence, and arteriosclerotic aneurysms. The risk of such diseases can be reduced by increasing the level of HDL cholesterol in the blood and/or decreasing the level of LDL cholesterol in the blood.

Renewed emphasis has been placed on lowering blood cholesterol, particularly the Low Density Lipoprotein (LDL) faction, the major reservoir of cholesterol in blood plasma. Many attempts have been made to lower the blood cholesterol level in individuals through dietary management, behavior modification, exercise, and drug therapy aimed at reducing or controlling the blood cholesterol levels.

The first recommendation in treating hypercholesterolemia is generally dietary intervention, whereby lipid intake has been restricted. Dr. Dean Omish et al. "Can Lifestyle Changes Reverse Coronary Heart Disease," The Lancet. vol. 336 (1990) and his ongoing program for reversing heart disease, has shown that complete elimination of dietary cholesterol and limiting fat content to less than ten percent of the daily caloric intake can effect a four percent regression of atherosclerotic plaque after five years when combined with stress management and aerobic exercise. This strict vegetarian diet (free of meat, fish, chicken, vegetable oils and all dairy fat products) is unrealistic for most individuals.

Dietary supplements have offered some promise in the fight against hypercholesterolemia. For example, brans, psylliums, guar gum, lecithins, whey, red wines, fish oils and ginseng root extract have been reported to reduce high blood cholesterol or its consequences. The mechanisms are varied and include cholesterol sequestering, chelating, entrapment, and oxidation inhibition. Such regimens generally affect only less than ten percent reduction in blood cholesterol. None of these dietary interventions have been shown to arrest or cure atherosclerosis or other high blood cholesterol associated diseases.

The most severe dietary intervention has been reported in U.S. Pat. No. 5,032,608 where it describes intravenous feeding of a mixture of biologically active levorotatory amino acids which replace all other feedings except water. A functionally equivalent fat free dietary composition is described in U.S. Pat. No. 5,106,836 which discloses a method for preparing enteral food products with a modified fat free total parenteral nutritional amino acid formulation. The prepared foods, when digested and absorbed, deliver an amino acid profile into the blood having hypocholesterolemic properties. It is reported that a significant reduction in total plasma cholesterol and regression of atherosclerosis is shown if the diet is maintained.

Other attempts to lower serum cholesterol levels have been directed to the development of various pharmaceutical preparations. For example, a bacterial cell product has been reported in U.S. Pat. No. 4,797,278 to lower blood cholesterol and/or triglyceride levels by having the ability to adhere to intestinal epithelial cells of the intestine thereby optimizing the conditions for colonization of beneficial bacteria in the appropriate areas in the intestine.

U.S. Pat. No. 5,114,963 describes a method of reducing atherosclerotic disease by reducing serum levels of lipoprotein by the administration of N,S-diacryl-L-cystein. Methods of treating myocardial damage have also centered on the intravenous injection of compounds in combination with fibrinolytic enzymes to digest or dissolve blood thrombosis, lysing fibrin clots and re-establishing and maintaining perfusion of ischemic tissue. This use of enzymes, whose amelioration of the consequence of atherosclerosis is described in U.S. Pat. No. 5,028,599, does not lower blood cholesterol concentration.

In view of the above failures of dietary, enzyme therapy, and lifestyle interventions, other means have been sought to lower blood cholesterol, reverse arterial plaque deposits, and otherwise mitigate the effects of high blood cholesterol on other tissue. The lowering of cholesterol with hypocholesterolemic drugs, cholesterol anti-absorption drugs (e.g. melinamide, thioesters, substituted urea and thiourea), certain cyclodextrins that act as substitute apoproteins cholesterol carriers, and cholesterol biosynthesis limiting drugs to reduce the risk of coronary heart disease is supported by convincing evidence to have a causal association with the lowering of blood cholesterol levels. These drugs and their adverse side effects such as liver impairment are well described in the Physicians' Desk Reference Medical Economics Company Oradell, N.J.

None of the above drugs alone or in combination, have been shown to significantly lower blood cholesterol and reduce atherosclerosis plaque without requiring concurrent severe restriction on ingestion of lipids. To date, there remains a dearth of effective and safe drugs which would prevent and treat high cholesterol without any adverse side-effects. Therefore, an object of the present invention is to provide a safe and effective treatment for diseases associated with high cholesterol levels without the associated side effects and severe life style restrictions.

### The Role of Chromium

Dietary supplementation of chromium to normal individuals has been reported to lead to improvements in glucose tolerance, serum lipid concentrations, including high-density lipoprotein cholesterol, insulin and insulin binding (Anderson, *Clin. Psychol. Biochem.* 4;31-41,1986). Supplemental chromium in the trivalent form, e.g. chromic chloride, is associated with improvements of risk factors associated with adult-onset (Type 2) diabetes and cardiovascular disease.

Chromium is a nutritionally essential trace element. The essentiality of chromium in the diet was established in 1959 by Schwartz, as cited in *Present Knowledge in Nutrition,* page 571, fifth edition (1984, the Nutrition Foundation, Washington, DC). Chromium depletion is characterized by the disturbance of glucose, lipid and protein metabolism and by a shortened lifespan. Chromium is essential for optimal insulin activity in all known insulin-dependent systems (Boyle et al., *Southern Med. J.* 70:1449-1453, 1977). Insufficient dietary chromium has been linked to both maturity-onset diabetes and to cardiovascular disease.

The principal energy sources for the body are glucose and fatty acids. Chromium depletion results in biologically ineffective insulin and compromised glucose metabolism. Under these conditions, the body must rely primarily on lipid metabolism to meet its energy requirements, resulting in the production of excessive amounts of acetyl-CoA and ketone bodies. Some of the documented acetyl-CoA is diverted to increased cholesterol biosynthesis, resulting in hypercholesterolemia. Diabetes mellitus is characterized in large part by glycosuria, hypercholesterolemia, and often ketoacidosis. The accelerated atherosclerotic process seen in diabetics is associated with hypercholesterolemia (Boyle et al., *supra*.).

Chromium functions as a cofactor for insulin. It binds to the insulin receptor and potentiates many, and perhaps all, of its functions (Boyle et al., *supra.*). These functions include, but are not limited to, the regulation of carbohydrate and lipid metabolism. (*Present Knowledge in Nutrition, supra,* at p. 573-577). The introduction of inorganic chromium compounds *per se* into individuals is not particularly beneficial. Chromium must be converted endogenously into an organic complex or must be consumed as a biologically active molecule. Only about 0.5% of ingested inorganic chromium is assimilated into the body (*Recommended Daily Allowances,* Ninth Revised Edition, The National Academy of Sciences, page 160, 1980). Only 1-2% of most organic chromium compounds are assimilated into the body.

U.S. Patent No. Re. 33,988 discloses that when selected essential metals, including chromium, are administered to mammals as exogenously synthesized coordination complexes of picolinic acid, they are directly available for absorption without competition from other metals. This patent describes a composition and method for selectively supplementing the essential metals in the human diet and for facilitating absorption of these metals by intestinal cells. These complexes are safe, inexpensive, biocompatible, and easy to produce. These exogenously synthesized essential metal coordination complexes of picolinic acid (pyridine-2-carboxylic acid) have the following structural formula: 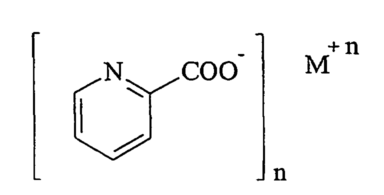 wherein M represents the metallic cation and n is equal to the cation's valence. For example, when M is Cr and n=3, then the compound is chromic tripicolinate. Other chromium picolinates disclosed include chromic monopicolinate and chromic dipicolinate.

The U.S. Recommended Daily Intake (RDI) of chromium is 120 µg. U.S. Patent No. 5,087,623 describes the administration of chromic tripicolinate for the treatment of adult-onset diabetes in doses ranging from 50 to 500 µg. International Patent Application No. WO96/35421 discloses the use of high doses of chromic tripicolinate (providing 1,000-10,000 µg chromium/day) for reducing hyperglycemia and stabilizing the level of serum glucose in humans with Type 2 diabetes. U.S. Patent Nos. 5,789,401 and 5,929,066 disclose a chromic tripicolinate-biotin composition and its use in lowering blood glucose levels in humans with Type 2 diabetes.

U.S. Patent Nos. 5,087,623; 5,087,624; and 5,175,156 disclose the use of chromium tripicolinate for supplementing dietary chromium, reducing hyperglycemia and stabilizing serum glucose, increasing lean body mass and reducing body fat, and controlling blood serum lipid levels, including the lowering of undesirably high blood serum LDL-cholesterol levels and the raising of blood serum High Density Lipid (HDL)-cholesterol levels, the so-called "good" cholesterol. U.S. Patent Nos. 4,954,492 and 5,194,615 describe a related complex, chromic nicotinate, which is also used for supplementing dietary chromium and lowering serum lipid levels. Picolinic acid and nicotinic acid are position isomers having the following structures:

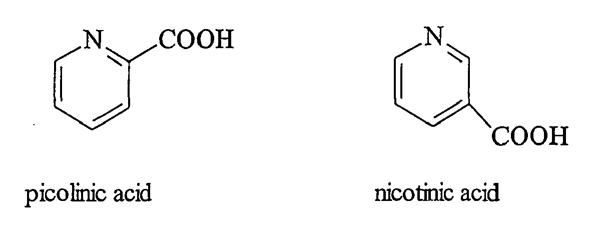

Nicotinic acid and picolinic acid form coordination complexes with monovalent, divalent and trivalent metal ions and facilitate the absorption of these metals by transporting them across intestinal cells and into the bloodstream. Chromium absorption in rats following oral administration of CrCl₃ was facilitated by the non-steroidal anti-inflammatory drugs (NSAIDs) aspirin and indomethacin (Davis et al., *J*. *Nutrition Res.* 15:202-210, 1995; Kamath et al., *J*. *Nutrition* 127:478-482, 1997). These drugs inhibit the enzyme cyclooxygenase which converts arachidonic acid to various prostaglandins, resulting in inhibition of intestinal mucus formation and lowering of intestinal pH which facilitates chromium absorption.

U.S. Patent 4.315,927 discloses that when selected essential metals are administered to mammals as exogenously synthesized coordination complexes of picolinic acid, they are directly available for absorption without competition from other metals. These complexes are safe, inexpensive, biocompatible and easy to produce.

Biotin is the prosthetic group for a number of carboxylation reactions, the most notable being pyruvate carboxylase which is involved in gluconsogenesis and replenishment of the citric acid cycle, and acetyl CoA carboxylese which plays a rule in fatty acid biosynthesis. The safe and adequate recommended daily intake of biotin is 100-300 µg, although no side effects or toxicities were noted in previous clinical studies with oral biotin intakes of up to 200 mg daily (Mock at al, in *Present Knowledge in Nutrition,* seventh edition, Ziegler, E. et al, eds., ILSI Press, Washington, DC, 1996, pp. 220-235). Supranutritional doses of biotin have been shown to have therapeutic utility in the treatment of various disease states such as diabetes. High-dose oral or parenteral biotin, for example, has been shown to improve oral glucose tolerance in diabetic KK mice (Reddi et al., *Life Sci.,* 42;1323-1330, 1988), rats made diabetic by injection with streptozotocin (Zhang et al., 16th international Congress of Nutrition, Montreal, 1997. abstract book, p. 264) and in pre-diabetic Otsuka Long-Evans Tokushima Fatty rats (Zhang et al., *J. Nutr. Sci. Vitaminol.* 42:517-526, 1996).

in a clinical study, Coggeshall et al. (*Ann. N.Y. Acad. Sci.,* 447:387-392, 1985) demonstrated that a daily oral dose of hintin of 16 mg lowered fasting plasma glucose levels in Type I diabetics in whom insulin injections had been temporarily discontinued. Maebashi et al. (*J*. *Clin. Biochem. Nutr.* 14:211-218, 1993) showed that admmistration of 3 mg biotin three times per day to poorly-controlled type 2 diabetics resulted in improved pancreatic beta cell function as evidenced by the fact that fasting insulin levels did not decline in biotin-treated subjects despite the sharp decline in glucose levels.

There is a constant need for effective treatments for lowering LOL and triglyceride levels while increasing HDL levels in a subject. The present invention addresses this need by providing a safe, inexpensive, drug-free therapeutic agent. A method of reducing and reversing dyslipidemia without patient side effects would present a substantial advancement in prevention and treatment of a variety of disease states.

### Summary of the Invention

In a first aspect, the present invention relates to the use of a chromium complex and biotin in the manufacture of a medicament for the treatment of dyslipidemia.

In another aspect, the present invention relates to the use of a chromium complex in the manufacture of a medicament for use in combination with biotin for the treatment of dyslipidemia.

In a further aspect, the present invention relates to the use of biotin in the manufacture of a medicament for use in combination with a chromium complex for the treatment of dyslipidemia,

In one embodiment the amount of chromium complex administered per day is between about 25 and 2,000 micrograms, preferably between about 300 and 1,000 micrograms per day.

In one embodiment the amount of biotin administered per day is between about 25 µg and 20 mg, preferably between about 150 µg and 5 mg per day.

The chromium complex of the present invention may include chromium picolinate, chromic tripicolinate, chromium nicotinate, chromic polynicotinate, chromium chloride, chromium histidinate, or chromium yeasts.

Preferably, the chromium complex is in a pharmaceutically acceptable carrier. Similarly, it is preferred that the biotin likewise is in a pharmaceutically acceptable carrier.

Optionally, the chromium complex and biotin are orally administered. However, in some aspects of the invention, the chromium complex and hintin are parenterally administered.

In yet another aspect of the invention, certain chelating agents may be added to facilitate absorption of the chromium complex. In one aspect of the invention, picolinic acid is administered to an individual. In another aspect, nicotinic acid is administered to an individual. In still another aspect, both picolinic and nicotinic acid are administered to an individual in order to treat dyslipidemia.

### Brief Description of the Drawings

Figure 1 illustrates the insulin signaling cascade.

Figure 2 is a graph demonstrating 2-deoxyglucose uptake with chromium picolinate and biotin.

Figure 3 is a graphic representation of glycogen synthesis in human skeletal muscle culture when incubated with chromium picolinate and biotin.

Figure 4A is a bar graph depicting glycogen synthesis.

Figure 4B is a bar graph illustrating gene expression (mRNA).

Figure 5 is a chart depicting the study design for evaluation of the effect of chromium and biotin on insulin sensitivity in rats.

Figure 6 Is a bar graph representing the results of co-administration of chromium and biotin on glucose disposal in a rat model

Figure 7 is a bar graph illustrating the effects of chromium picolinate on fasting plasma insulin levels in obese rats at baseline and end of study.

Figure 8A is a graphic depiction of the results of the intraperitoneal glucose tolerance test in obese rats and the effects of chromium picolinate on glucose tolerance.

Figure 8B demonstrates the insulin response observed after treatment with chromium picolinate.

Figure 9 is a bar graph demonstrating the effects an insulin sensitivity in obese rats as compared to lean rats with the administration of chromium picolinate versus the control.

Figure 10 is a line graph representing the effects observed over time on the cholesterol levels of rat models treated with a variety of chromium and biotin protocols.

Figure 11 is a bar graph depicting the change of cholesterol over time in various treatment protocols involving the administration of high and low doses of chromium and biotin, alone or in combination.

Figure 12A is a bar graph demonstrating the HDL-cholesterol profile in JCR rats treated with chromium picolinate.

Figure 12B is a bar graph detailing the cholesterol/HDL ratio in JCR rats treated with chromium picolinate.

Figure 13 is a bar graph illustrating the HDL/cholesterol ratio in treated and untreated JCR rats.

Figure 14 is a bar graph representing the change in HDL levels over time of JCR rats that have been administered various combinations of chromium and biotin.

Figure 16 is a line graph charting the HOL profils of test animals administered various doses of chromium and biotin, either alone or in combination.

Figure 16 is a line graph detailing the change in triglyceride levels over time in rats treated with chromium and biotin, either alone or in combination.

Figure 17 is a bar graph illustrating the change in triglyceride profile in rats administered various doses of chromium, biotin, or both.

Figure 18 is a bar graph illustrating the effect of chromium picolinate and biotin added beverage an glycosylated hemoglobin levels.

Figure 19 is a bar graph demonstrating the effect of chromium picolinate and biotin added beverage an blood glucose.

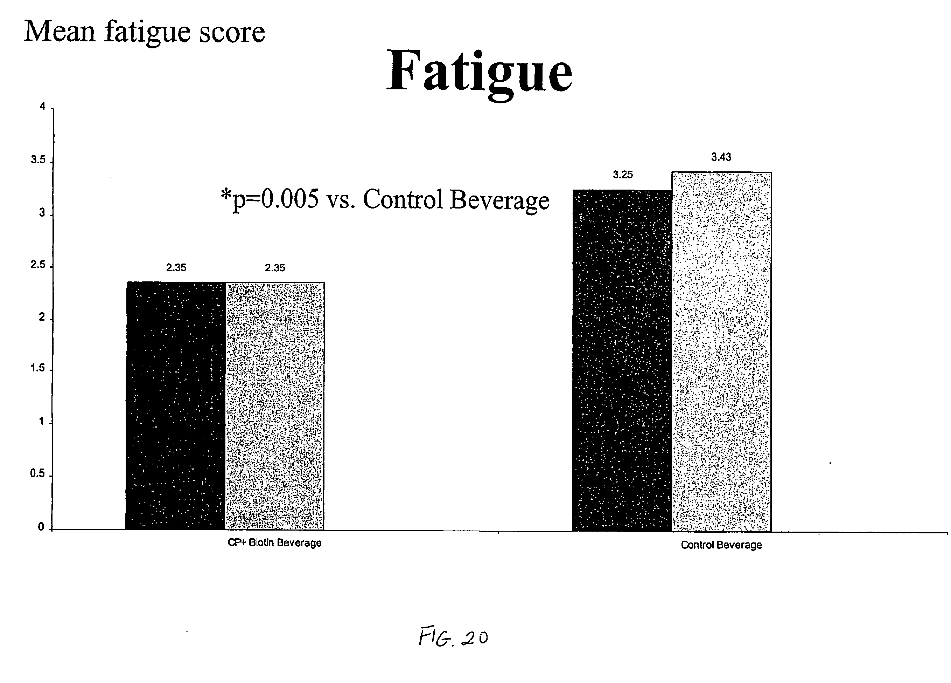

Figure 20 is a graphic representation of the effect of chromium picolinate and biotin on fatigue.

### Detailed Description of the Preferred Embodiment

The disclosed invention relates to methods for the treatment of dyslipidemia.

The terminology used in the description presented herein is not intended to be interpreted in any limited or restrictive manner, simply because it is being utilized in conjunction with a detailed description of certain specific embodiments of the invention. Furthermore, embodiments of the invention may include several novel features, no single one of which is solely responsible for its desirable attributes or which is essential to practicing the invention herein described. As used herein, the term "chromium complexes" or "chromium complex" includes, without limitation, chromium picolinate, chromic tripicolinate, chromium nicotinate, chromic polynicotinate, chromium chloride, chromium histidinate, and chromium yeasts.

A primary basis of the present invention is the novel and unexpected discovery that the co-administration of an effective dose of a chromium complex in combination with biotin produces a synergistic improvement in insulin sensitivity. The co-administration of chromium and biotin can facilitate the treatment and recovery of individuals suffering from a variety of medical conditions such as dyslipidemia. Moreover, the administration of a chromium complex in combination with an effective dose of biotin provides an effective reduction of hypercholesterolemia and increase in HDL cholesterol in the blood. This reduction is markedly greater than what would be expected when either component is administered alone, this indicating a synergistic effect.

Insulin resistance is a key pathogenic parameter of Type 2 diabetes, and clinical interventions that improve insulin sensitivity are considered cornerstones in the management of the disease. In addition, the relationship of insulin resistance to cardiovascular disease and its associated risk factors has been well established over the past few years. Therefore, with the recent release of numerous medications, current treatment of Type 2 diabetes is aimed toward achieving "clinical insulin sensitization." This concept is based on the established clinical goal of lowering blood glucose in an effort to reduce microvascular complications (i.e. eye, kidney, and nerve disease) with minimal endogenous insulin stimulation or the lowest exogenous insulin dosing possible. Because of such a clinical concept, combination therapy for treatment of Type 2 diabetes is now considered the standard of care in clinical management. Combinations of pharmacologic agents (such as sulfonylureas/metformin, sulfonylureas/glitazones, and metformin/glitazones) are highly effective pharmacologic interventions that appear to lower both glucose and insulin levels. Further, there is evidence that triple drug therapy (e.g. sulfonylureas/metformin/glitazones) can lower clinical glycemia in addition to lowering insulin levels. Because of the success of these clinical formulations, pharmacologic agents that have a clinical effect to both lower glucose and improve insulin action will be considered very favorably in the future management of Type 2 diabetes.

In contrast to pharmacologic therapy, nutritional intervention is a very attractive approach to treatment of insulin resistance and therefore, glucose control of Type 2 diabetics, in this regard, there is strong evidence to suggest that supplemental chromium complexes such as chromium picolinate (CrPic) may favorably improve insulin sensitivity and glycemic control in human trials. Specifically, this has been demonstrated in a study of Chinese diabetics, where CrPie significantly lowered glucose and insulin levels. Anderson et al. *Elevated intakes of supplemental chromium improve glucose and insulin variables in individuals with type 2 diabetes*. Diabetes, 48: 1786-1791 (1997). Recently, it has been demonstrated that CrPic at 1000 µg per day can enhance insulin sensitivity in a cohort of subjects representing the obese, pre-diabetic human population. Cefalu, W.T. et al, *Effects of chromium picolinate on insulin sensitivity in vivo.* J. Trace Elem. Exp. Med. 12: 71-83 (1999). Similarly, CrPic has a very robust effect on improving insulin sensitivity and lipid profiles in an obese, hyperinsulinemic, insulin resistant rodent model. Wang et al. *Chromium picolinate enhances insulin sensitivity in an animal model for the metabolic syndrome: the obese, insulin resistant JCR:LA-corpulent rat.* Diabetes; In press (abstract to be presented at the Annual Meeting of the ADA. June 2000) (2000). This strongly suggests that subjects with the components of "Syndrome X" (i.e. central obesity, dyslipidemia, and insulin resistance) may favorably respond to chromium supplementation.

In addition to monotherapy treatments with a chromium complex such as chromium picolinate, recent research strongly suggests that other nutrients, such as biotin, can enhance the effect of chromium observed on glucose uptake. Specifically, the combination of a chromium complex with biotin greatly enhances glucose uptake in a human skeletal muscle culture. Without being limited to a particularly theory, it is believed that the cellular mechanism by which a chromium complex may enhance insulin sensitivity is improved by enhancing glycogen synthesis. The importance of this observation in understanding the mechanism by which chromium enhances insulin sensitivity is heightened by the fact that the aspect of insulin resistance that has been the most described is the inefficient glucose uptake and utilization in response to insulin sensitivity in insulin sensitive tissues. This is represented by a reduction in the insulin-stimulated storage of glucose as glycogen in both muscle and liver.

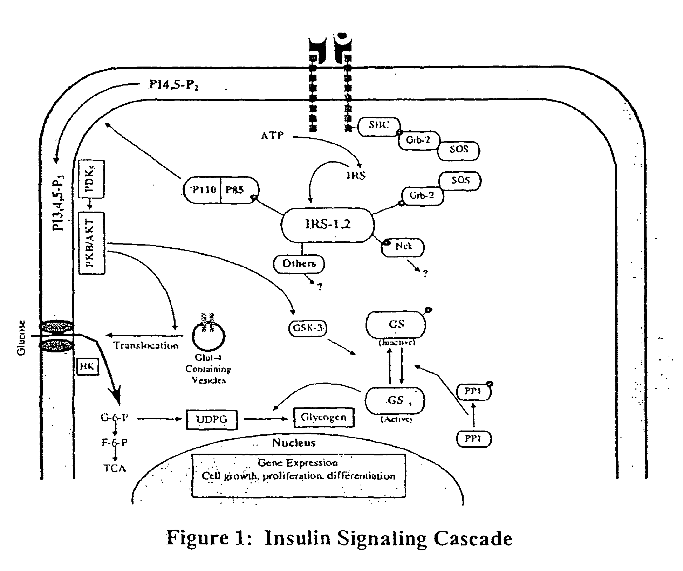

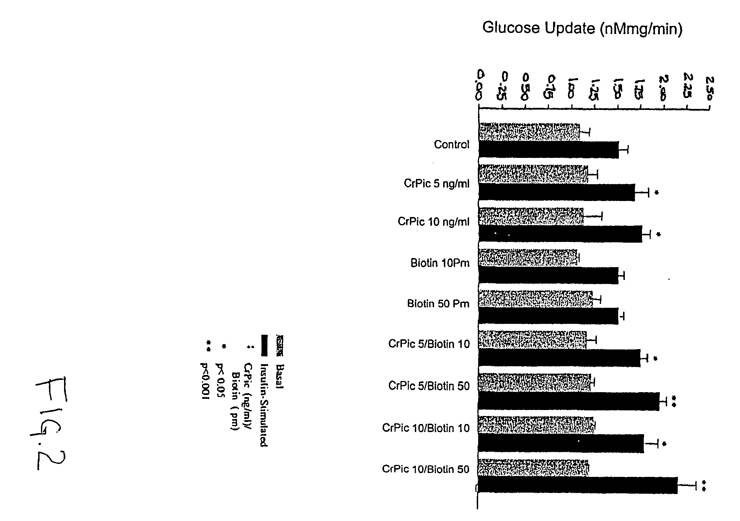

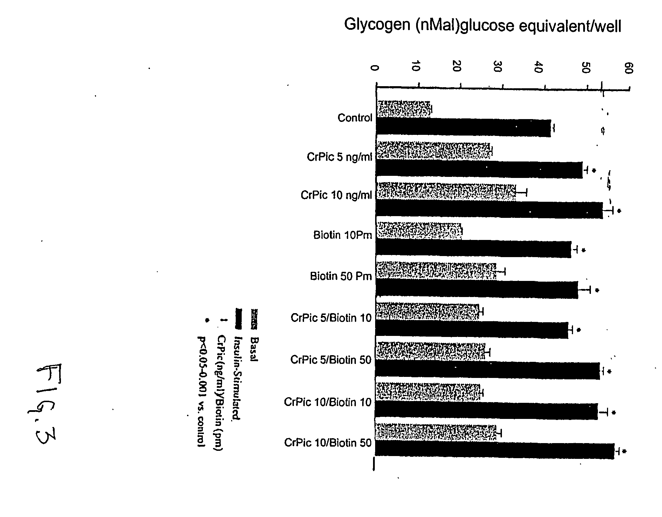

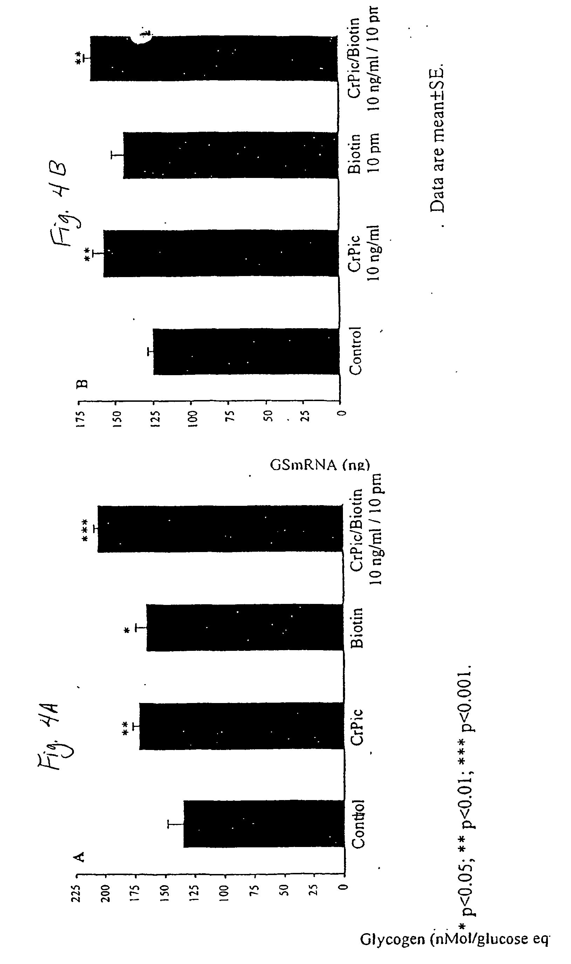

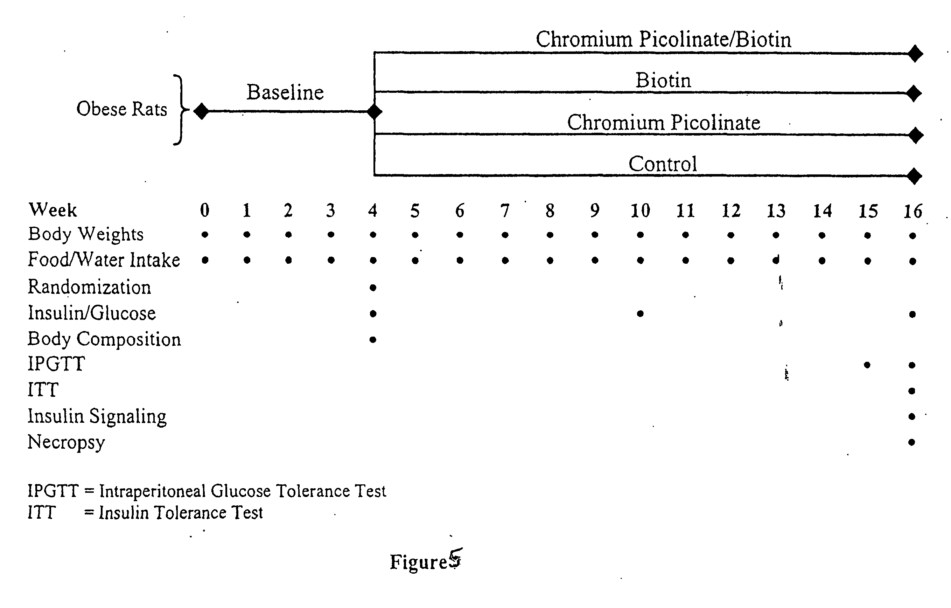

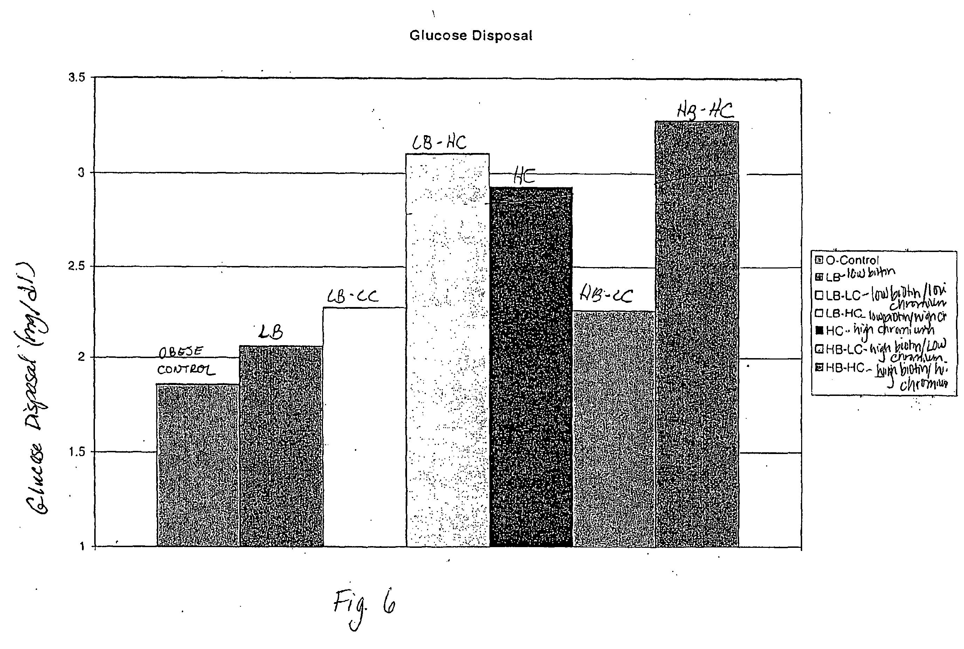

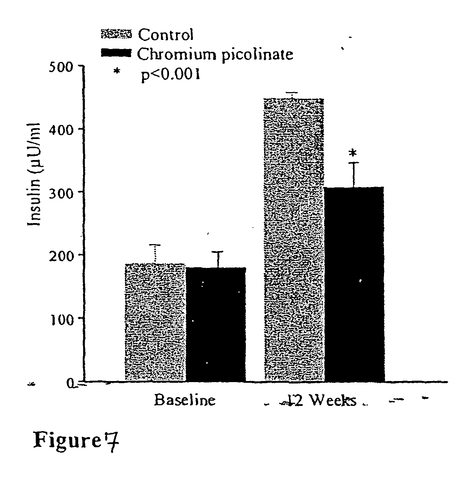

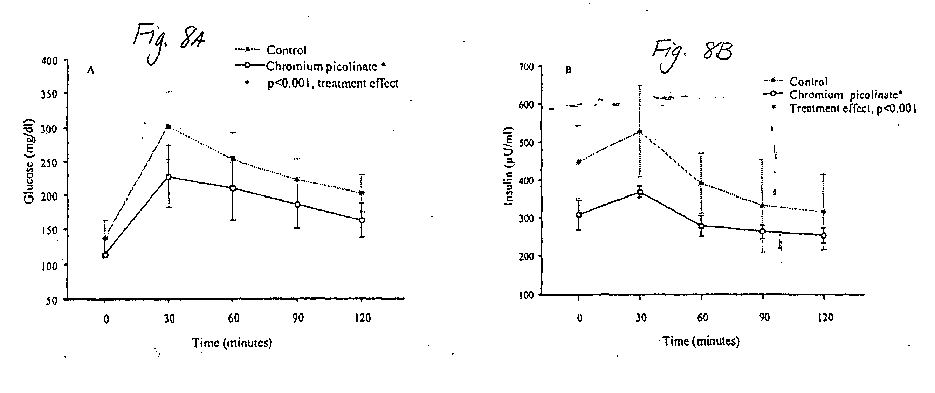

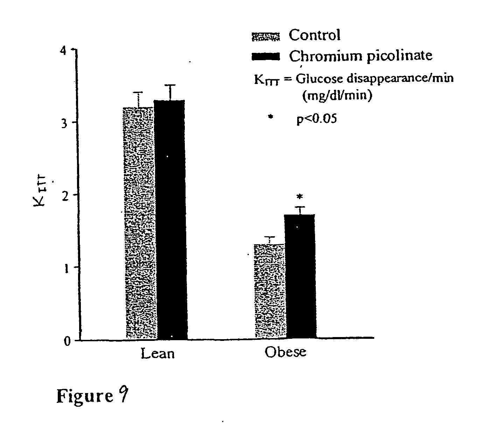

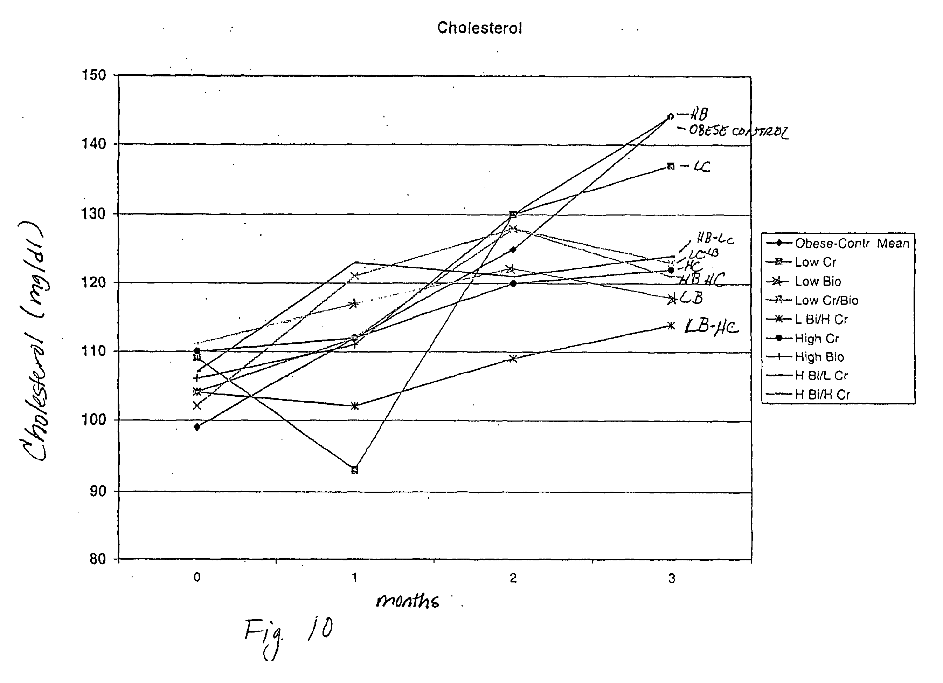

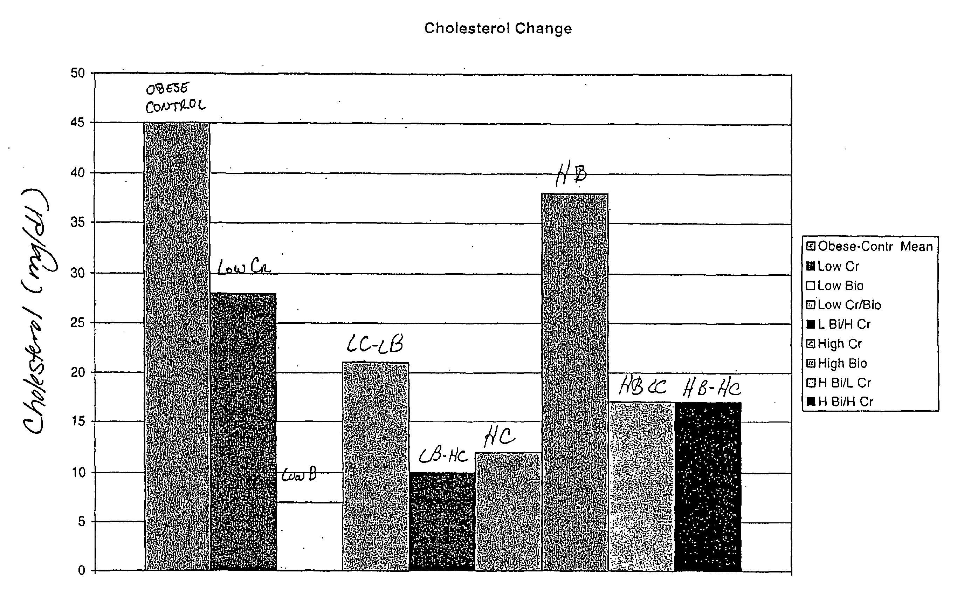

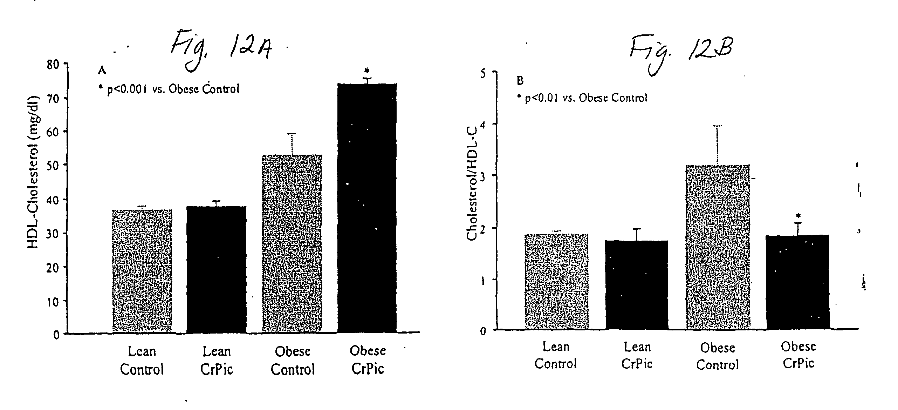

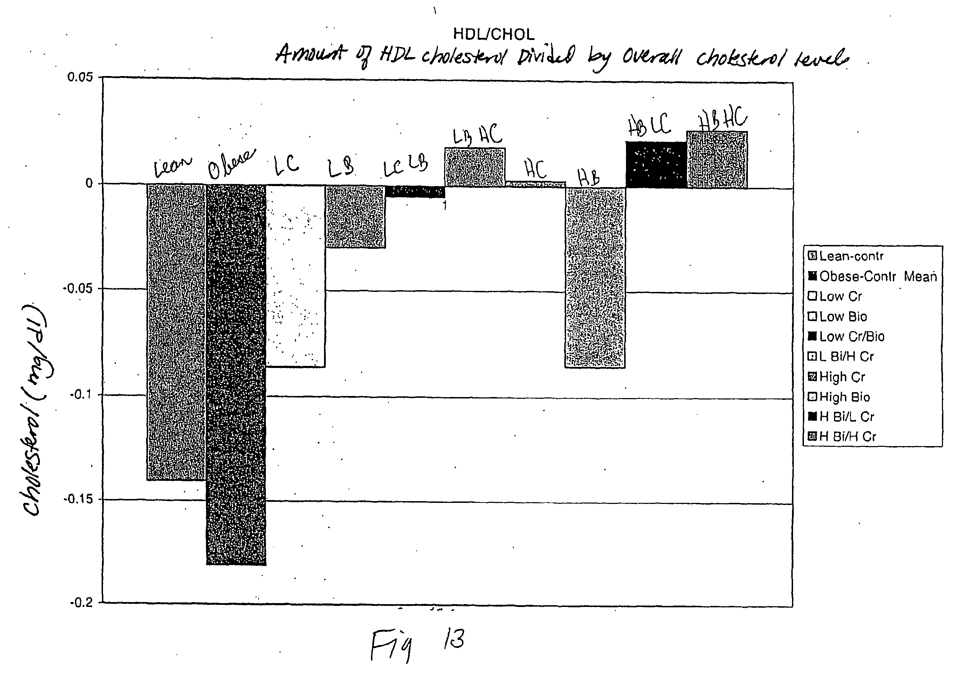

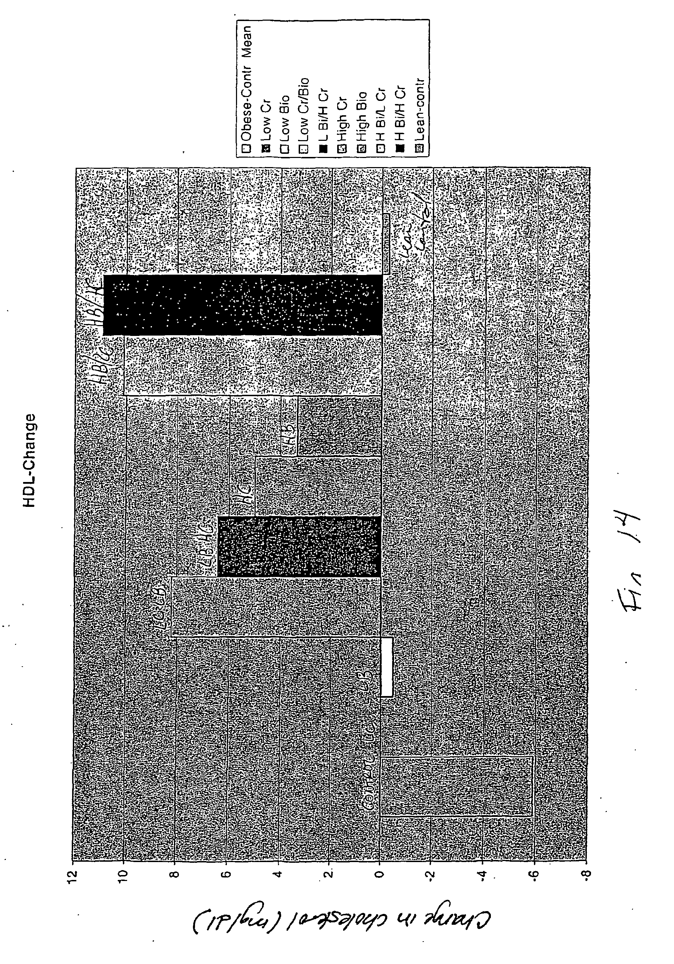

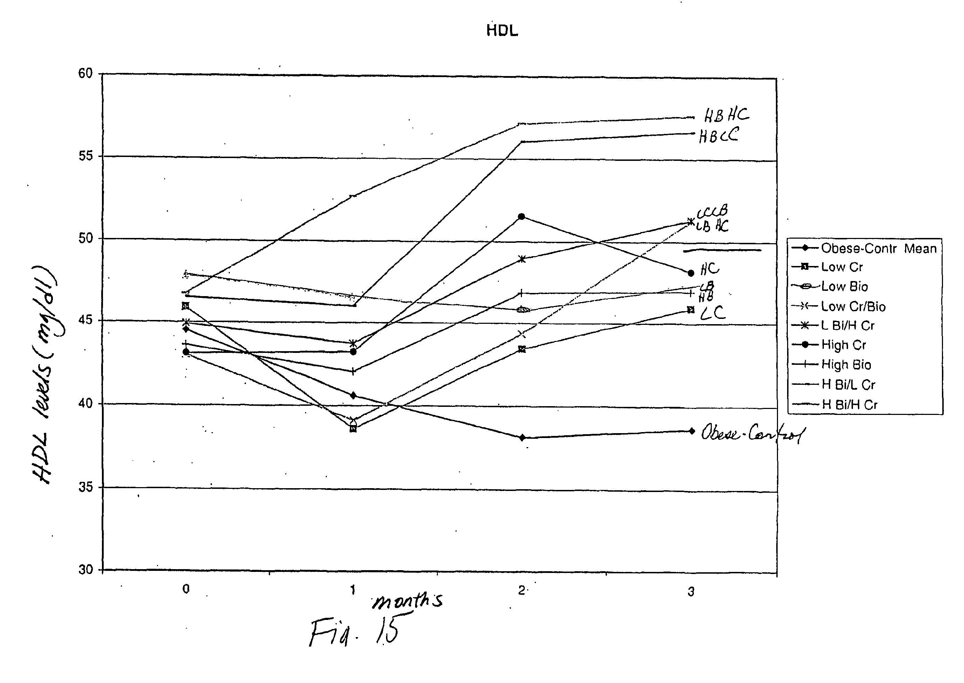

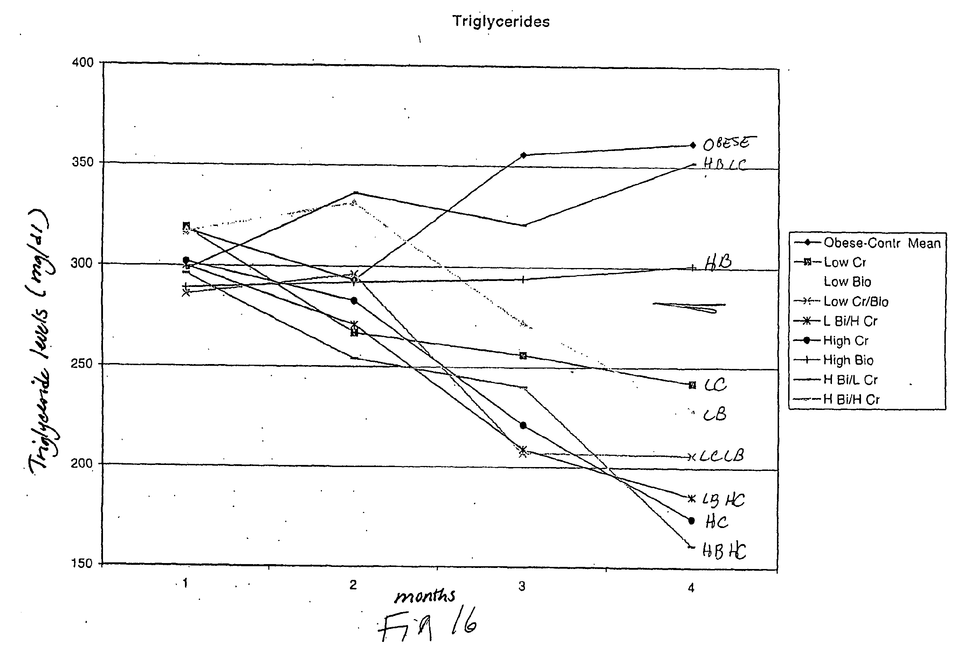

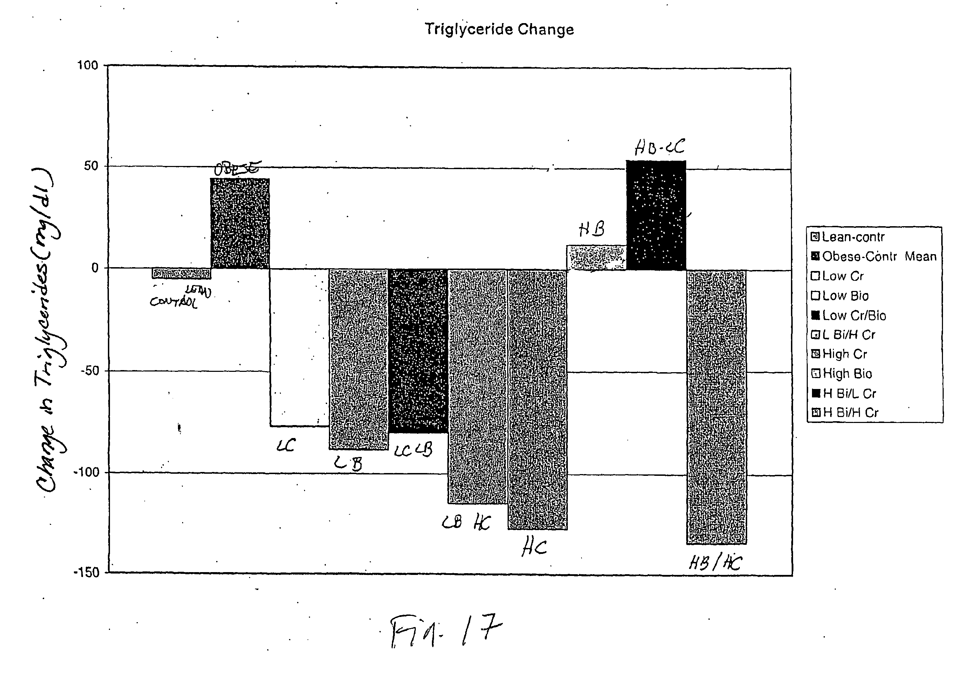

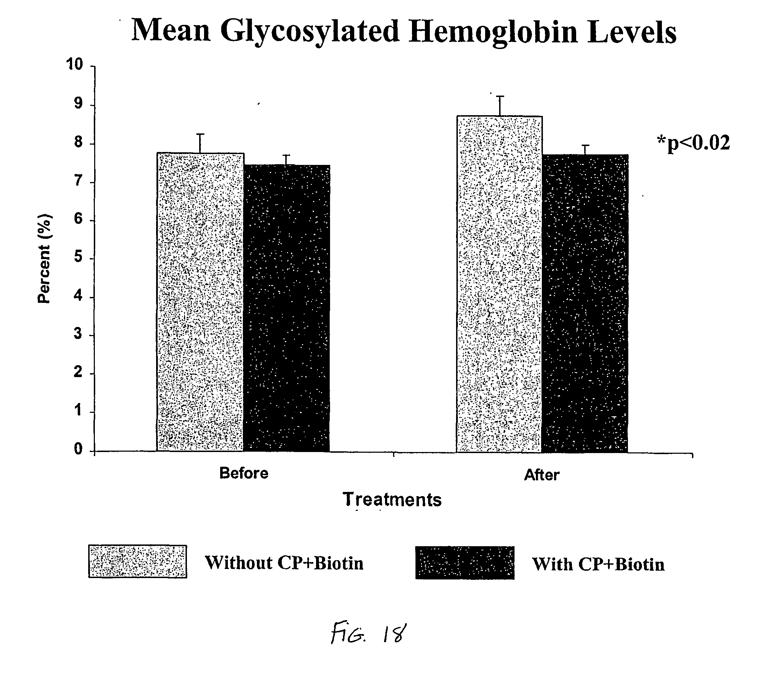

The specific cellular defects that characterize insulin resistance have not been fully characterized. However, Figure 1 outlines currently accepted pathways in the insulin signaling cascade and specifically outlines pathways involved in glucose transporter (Glut-4) translocation and glucogen synthesis. These two cellular parameters are altered in insulin resistant states and overcoming these defects leads to an increase in insulin sensitivity. In this regard, the present invention is directed to exploring the role of biotin and chromium complexes in enhancing cellular signaling, leading to enhanced target insulin both *in vitro* and *in vivo.*

The present invention contemplates using chromium complexes in combination with biotin to achieve a beneficial reduction in LDL and/or increase in HDL cholesterol levels in the blood. The compounds of the present invention can be administrated to an individual separately or as a single composition. Advantageously, an individual is administered a pharmaceutically effective dose of a chromium complex such as chromium picolinate. In one embodiment, the biotin is administered substantially simultaneously. In an alternative embodiment, the chromium complex is administered first and then the biotin is added second. In yet another embodiment, the biotin is administered first. If administered separately, the compounds should be given in a temporally proximate manner, e.g. within a twenty-four hour period, such that the reduction of hypercholesterolemia is enhanced. More particularly, the compounds may be given within one hour of each other. The administration can be by any of the methods of administration described below or by drug delivery methods known by one of skill in the art.

The synthesis of chromic picolinates is described in U.S. 5,087,623. Biotin and chromium complexes such as chromium tripicolinate are commercially available from health food stores, drug stores and other commercial sources. In order to lower LDL cholesterol levels and/or increase HDL levels, it is anticipated that the dosage range of chromium administered to an individual will be at least about 26 µg/day. Preferably, the amount of chromium will be between about 25 and 2,000 µg/day. More preferably, the amount of chromium is between about 300 and 1,000 µg/day. Most preferably, the amount of chromium is between about 400 and 1,000 µg/day. In a particularly preferred embodiment, the amount of chromium is between about 600 and 1,000 µg/day, With regard to the biotin component of the combination therapy, the daily dosage is at least 25 µg. Preferably, the amount of biotin is between about 25 µg and 20 mg par day. More preferably, the daily dosage of biotin is between about 150 µg to 10 mg. Most preferably, the daily dose of biotin is between about 300 µg and 5 mg. Note that these doses are based on a 70 kg adult human, and that the dose can be applied on a per-kilogram basis to humans or animals of different weights.

While the chromium complexes aid in the absorption of chromium by intestinal cells, in some embodiments, uncomplexed chelating agents are advantageously included in the compositions to facilitate absorption of other ingested chromium as well as other metals including, but not limited to, copper, iron, magnesium, manganese, and zinc. Suitable chelating agents include picolinic acid, nicotinic acid, or both picolinic acid and nicotinic acid. Thus, the compositions of the disclosed invention are readily absorbable form of chromium which also facilitate absorption of other essential metals in the human diet

The chromium complexes of the disclosed invention have the same uses as described for chromic tripicolinate in U.S. Patent Nos. 5,087,623, 5,087,024 and 5,174,156, namely supplementing dietary chromium, lowering blood glucose levels in diabetics, lowering serum lipid levels and increasing lean body mass. Additionally, the chromium picolinate of the present invention act to treat symptoms associated with diabetes.

Advantageously, the chromium complexes are synthetic. The synthesis and use of chromium picolinates, for example, is described in U.S. Patent Nos. Re 33,988 and 5,087,623, Chromic tripicolinate is available from health food stores, drug stores and other commercial sources. The synthesis and use of chromic polynicotinate is described in U.S. Patent No.5,194,615.

The chelating agents such as picolinic acid and nicotinic acid are available from many commercial sources, including Sigma-Aldrich (St. Louis, MO) (picolinic acid; catalog No. P5503; nicotinic acid; catalog No. PN4126). Preferably, the ratio of the chromium complex to the chelating agent from about 10:1 to about 1:10 (w/w), more preferably from about 6:1 to about 1:5 (w/w). Alternatively, the molar ratio of chromium complex to the uncomplexed chelating agent is preferably 1:1, and may be from about 5:1 to about 1:10.

For oral administration, the chromium complex and biotin may be provided as a tablet, aqueous or oil suspension, dispersible powder or granule, emulsion, hard or soft capsule, syrup, elixir, or beverage. Compositions intended for oral use may be prepared according to any method known in the art for the manufacture of pharmaceutically acceptable compositions and such compositions may contain one or more of the following agents: sweeteners, flavoring agents, coloring agents and preservatives. The sweetening and flavoring agents will increase the palatability of the preparation. Tablets containing chromium complex in admixture with non-toxic pharmaceutically acceptable excipients suitable for tablet manufacture are acceptable. Pharmaceutically acceptable means that the agent should be acceptable in the sense of being compatible with the other ingredients of the formulation (as well as non-injurious to the patient). Such excipients include inert diluents such as calcium carbonate, sodium carbonate, lactose, calcium phosphate or sodium phosphate; granulating and disintegrating agents, such as corn starch or alginic acid; binding agents such as starch, gelatin or acacia; and lubricating agents such as magnesium stearate, stearic acid or talc. Tablets may be uncoated or may be coated by known techniques to delay disintegration and absorption in the gastrointestinal tract and thereby provide a sustained action over a longer period of time. For example, a time delay material such as glyceryl monostearate or glyceryl distearate alone or with a wax may be employed.

Formulations for oral use may also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, such as peanut oil, liquid paraffin or olive oil. Aqueous suspensions may contain the chromium complex of the invention in admixture with excipients suitable for the manufacture of aqueous suspensions. Such excipients include suspending agents, dispersing or wetting agents, one or more preservatives, one or more coloring agents, one or more flavoring agents and one or more sweetening agents such as sucrose or saccharin.

Oil suspensions may be formulated by suspending the active ingredient in a vegetable oil, such as arachis oil, olive oil, sesame oil or coconut oil, or in a mineral oil such as liquid paraffin. The oil suspension may contain a thickening agent, such as beeswax, hard paraffin or cetyl alcohol. Sweetening agents, such as those set forth above, and flavoring agents may be added to provide a palatable oral preparation. These compositions may be preserved by an added antioxidant such as ascorbic acid. Dispersible powders and granules of the invention suitable for preparation of an aqueous suspension by the addition of water provide the active ingredient in admixture with a dispersing or wetting agent, a suspending agent, and one or more preservatives. Additional excipients, for example sweetening, flavoring and coloring agents, may also be present.

Syrups and elixirs, may be formulated with sweetening agents, such as glycerol, sorbitol or sucrose. Such formulations may also contain a demulcent, a preservative, a flavoring or a coloring agent.

The chromium complex preparations for parenteral administration may be in the form of a sterile injectable preparation, such as a sterile injectable aqueous or oleaginous suspension. This suspension may be formulated according to methods well known in the art using suitable dispersing or wetting agents and suspending agents. The sterile injectable preparation may also be a sterile injectable solution or suspension in a non-toxic parenterally-acceptable diluent or solvent, such as a solution in 1,3-butanediol. Suitable diluents include, for example, water, Ringer's solution and isotonic sodium chloride solution. In addition, sterile fixed oils may be employed conventionally as a solvent or suspending medium. For this purpose, any bland fixed oil may be employed including synthetic mono or diglycerides. In addition, fatty acids such as oleic acid may likewise be used in the preparation of injectable preparations.

The pharmaceutical compositions may also be in the form of oil-in-water emulsions. The oily phase may be a vegetable oil, such as olive oil or arachis oil, a mineral oil such as liquid paraffin, or a mixture thereof. Suitable emulsifying agents include naturally-occurring gums such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsions may also contain sweetening and flavoring agents.

The amount of chromium complex/biotin that may be combined with the carrier material to produce a single dosage form will vary depending upon the host treated and the particular mode of administration.

### EXAMPLES

The following examples teach the methods and compositions disclosed herein for improving insulin sensitivity, reducing cholesterol levels, treating post prandial hyperglycemia, and reducing the glycemic index of food through the administration of at least one chromium complex in concert with biotin.

### Reference EXAMPLE 1

### Effects of chromium and biotin on insulin sensitivity

To evaluate the potential cellular mechanism for CrPic's *in vivo* effect, the role of chromium was evaluated, as monotherapy and in combination with biotin, on glucose uptake in a human skeletal muscle culture ("HSMC"). The value of the HSMC has been reported as representative of an insulin sensitive target tissue and skeletal muscle is the major tissue for glucose disposal in humans. *See* Henry, R.R. et al. *Acquired defects of glycogen synthase activity in cultured human skeletal muscle cells: influence of high glucose and insulin levels.* Diabetes, 45: 400-407 (1996).

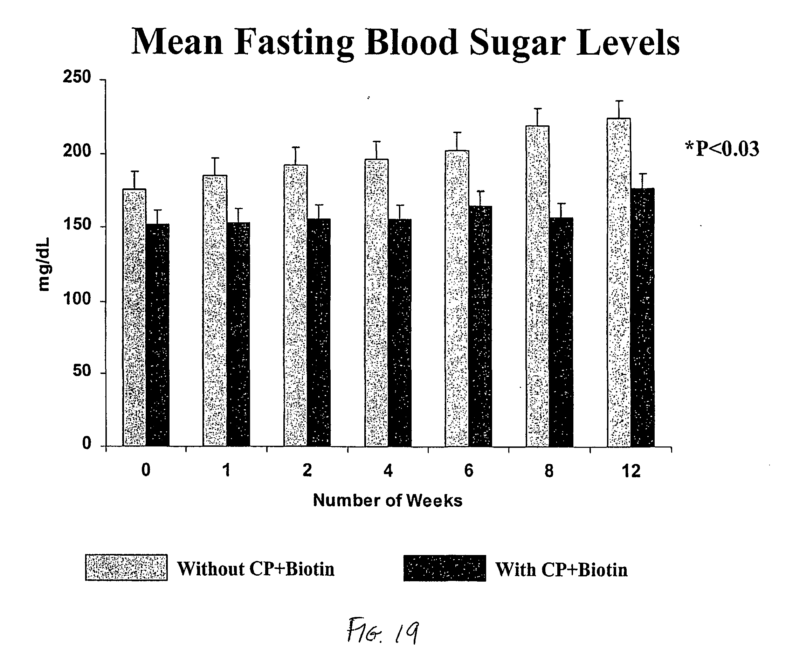

With specific regard to glucose uptake, we have demonstrated that chromium alone enhances glucose uptake in contrast to biotin alone. However, when the HSMC was incubated with both, a synergistic effect on glucose uptake was observed as illustrated in Figure 2. This observation was further extended to the assessment of glucogen synthesis. The human skeletal muscle cell line was plated and grown in the presence of CrPic, biotin, and combined CrPic/biotin. After incubation, cells were fasted and glycogen synthesis was analyzed at baseline and after insulin stimulation. Chromium and biotin increased glycogen synthesis both at basal and insulin-stimulated conditions as illustrated in Figure 3. Protein content of IRS-2 was also increased in CrPic + biotin combination.

### Reference EXAMPLE 2

### Evaluation of the cellular mechanism by which CrPic and biotin enhance glycogen synthesis

To evaluate the cellular mechanism by which CrPic and biotin enhance glycogen synthesis, glycogen synthetase ("GS") mRNA and glycogen synthesis were assessed. HSMC was incubated with CrPic at 10ng/ml, biotin at 10 pm, and both CrPic and biotin. As demonstrated, CrPic and biotin were again observed to enhance insulin stimulated glycogen synthesis as depicted in Figure 4A. When assessing gene expression, the combination of CrPic and biotin was observed to enhance GS mRNA (see Figure 4B). When evaluating GS mRNA levels, chromium increased gene expression of GS by 26%, biotin by 15%, and the combination by 33%. This strongly suggests a synergistic effect of chromium and biotin on GS gene expression.

The mechanism proposed is that CrPic and biotin affect the rate of gene transcription of enzymes (particularly GS) involved in mediating the biologic effects of insulin in human skeletal muscle.

### Reference EXAMPLE 3

### Effect of chromium and biotin on insulin sensitivity in an animal model

In order to test the effects of chromium and biotin, alone and in combination, on insulin sensitivity, a rodent model of "Syndrome X" was employed. Specifically, the JCR rat was utilized as it is a well-established model of obesity and insulin resistance. In addition, this model exhibits clinical components of the insulin resistance syndrome (e.g., dyslipidemia, central obesity).

After a baseline phase where accurate weights, body composition, and food intake were assessed, the animals were randomized to either control, CrPic alone, biotin alone, or CrPic/Biotin (See Figure 5). Animals were weighed weekly and nutrients were given via daily water feeding at a specified dose/kg body weight. A 12-week period of daily treatment of animals was accomplished.

### Methods

At specified time points during the study, glucose and insulin assessments were made. Specific assessments of carbohydrate metabolism were determined using an intraperitoneal glucose tolerance test and an insulin tolerance test. Animals were studied at 6 and 12 weeks with randomization. The specific metabolic testing was as follows:

### Intraperitoneal Glucose Tolerance Test (IPGTT)

Following baseline glucose and insulin measurements, 1.5 mg D₅₀W per gram of body weight were given intraperitoneally. Tailsticks were initiated at 30, 60, 90, and 120 minutes following administration of glucose. The areas under curve for insulin and glucose were assessed. Eight animals in each group at 12 weeks were studied. The results of the IPGTT are reflected in Table 1. Table 1 details the amount of glucose disposal for animals treated with CrPic and/or biotin at various dosages. Note, "L" represents the lean control; "O-contr" are the obese control rats; "LB" connotes low biotin treatments; "HB" represents high biotin treatments; "LC" and "HC" represent low and high doses of chromium, respectively. Figure 6 is a graphic representation of the results of the IPGTT studies.

**Table 1: Glucose Disposal Data for the Chromium Picolinate + Biotin Animal Study**

| Rat # | Cage# | Treated | Body Weight | Glucose Disposal (mg/dl/min) | | |
|---|---|---|---|---|---|---|
| | 1 | L | 375 | 3.61 | O-Control | 1.8585 |
| | 2 | L | 443 | 4.424 | LB | 2.0615 |
| | 3 | L | 420 | 3.114 | LB-LC | 2.273333 |
| | 4 | L | 400 | 3.248 | LB-HC | 3.1005 |
| | 5 | L | 370 | 4.562 | HC | 2.928 |
| | 6 | L | 400 | 4.124 | HB-LC | 2.258667 |
| | 7 | L | 420 | 3.714 | HB-HC | 3.275333 |
| | 8 | L | 400 | 4.177 | | |
| | 9 | O-Contr | 751 | 2.005 | | |
| | 10 | O-Contr | 859 | 1.876 | | |
| | 11 | O-Contr | 900 | 1.632 | | |
| | 12 | O-Contr | 810 | 1.921 | | |
| | 13 | 9 LB | 866 | 2.21 | | |
| | 14 | 9 LB | 812 | 2.392 | | |
| | 15 | 10 LB | 867 | 1.876 | | |
| | 16 | 10 LB | 816 | 1.768 | | |
| | 17 | 11 LB LC | 816 | 2.55 | | |
| | 18 | 11 LB LC | 720 | 2.192 | | |
| | 19 | 12 LB LC | 953 | 2.078 | | |
| | 20 | 13LBHC | 955 | 3.12 | | |
| | 21 | 13 LB HC | 795 | 2.879 | | |
| | 22 | 14LBHC | 825 | 3.24 | | |
| | 23 | 14 LB HC | 837 | 3.163 | | |
| | 24 | 15 HC | 830 | 2.928 | | |
| | 25 | 17 HB | 758 | 1.92 | | |
| | 26 | 18 HB | 935 | 2.329 | | |
| | 27 | 18 HB | 856 | 1.879 | | |
| | 28 | 19HBLC | 815 | 2.34 | | |
| | 29 | 20 HB LC | 872 | 2.412 | | |
| | 30 | 20 HB LC | 840 | 2.024 | | |
| | 31 | 21 HB HC | 960 | 3.211 | | |
| | 32 | 22 HB HC | 820 | 3.311 | | |
| | 33 | 22 HB HC | 780 | 3.304 | | |
| | | | | | | |
| | 1 23 Cont | | 750 | | | |
| | 2 24 LC | | 720 | 2.334 | | |
| | 3 24 LC | | 750 | 2.109 | | |
| | 4 26 LB | | 835 | 1.875 | | |
| | 5 27 LB LC | | 748 | 2.31 | | |
| | 6 HB LC | | 722 | 2.2 | | |
| | 7 29 HC | | 855 | 3.25 | | |
| | 8 29 HC | | 795 | 3.224 | | |
| | 9 30 HC | | 705 | 3.011 | | |
| | 1030HC | | 786 | 2.821 | | |
| | 11 31 HB | | 700 | 1.772 | | |
| | 12 31 HB | | 788 | 1.993 | | |
| | 1334HBHC | | 727 | 3.04 | | |
| | 14 LC | | | 2.066 | | |

### Insulin Tolerance Test (ITT)

Following baseline glucose assessment, insulin (5 units per kg body weight) was given intravenously. Repeat glucose assessments occurred at 5, 15, and 30 minute intervals following insulin administration. The rate of glucose disappearance was measured. Again, eight animals in each group at 12 weeks were studied.

### Body Composition

Total body fat was determined at baseline and end of study. For this purpose, the animal was anesthetized with Metaphane, an inhaled gas that induces anesthesia in less than 30 seconds and has been found to be very well tolerated by rodents. The system to measure body composition is the TOBEC (total body electrical conductivity measuring device). The essential principle in this method is that an electromagnetic field is distorted as a direct function of the content of mineral containing tissues (i.e. lean tissue). The machine does not use radiation and poses no biological hazard. Once anesthetized, the rat was placed inside the TOBEC machine with no restraint, readings were taken, and measurements were performed.

### Skeletal Muscle Biopsy

At the end of the study, biopsies were made of the vastus lateralis muscle after insulin stimulation. The determination of glycogen content and gene expression studies were performed as follows:

*1. Glycogen Content of Skeletal Muscle:* Glycogen hydrolysis and glucose determination in the skeletal muscle biopsy were performed according to Gomez-Lechon with some modifications. Gomez-Lechon, M.J. et al. *A microassay for measuring glycogen in 96-well-cultured cells.* Anal. Biochem. 102: 344-352 (1980). After biopsy, the skeletal muscle cells were washed three times with PBS (pH 7.4). 100 nM insulin and 30 mM glucose were added to media (only SkBM), or added to the same volume of 0.9% NaCl as basal incubation for 2 hr. After extensive washes in ice-cold PBS, all liquid was removed and thereafter, 200 µl of 0.2M sodium acetate buffer, pH 4.8 was added to each well, sonicated using a high-intensity ultrasonic processor (VIR Sonic 60) at setting 7 for 20 sec. 50 µl/well aliquots of homogenates were taken for DNA assay, as described in Labarca, C. and Paigen, K. *A simple, rapid, and sensitive DNA assay procedure.* Anal. Biochem 102: 344-352 (1980). Amyloglucosidase was added to each well at final concentration at 500 mU/well. Plates were incubated at 40°C for 2 hr with shaking to prevent the sediment of glycogen-protein aggregates. The products of enzyme digestion were collected to 1.5 ml microcentrifuge tubes and centrifuged at 3000 rpm for 10 min. For the glucose assay, 50 µl/well aliquots of the above supernatants were transferred to a 96 well plate and 150 µl of assay solution (20 u/liter peroxidase, 10 u/liter glucose oxidase, and 1 g/liter ABST were added. Samples were incubated at room temperature in the dark for 30-40 minutes. The intensity of the color reaction was measured at 405 nm using a microplate reader. A blank of the reaction was performed by incubation of cell homogenates without glucoamylase; this value represented the free glucose content and was subtracted from the total glucose obtained after enzymatic hydrolysis. A standard curve was constructed with known amounts of rabbit liver glycogen and processed as test samples. The glycogen contents were expressed as nM glucose equivalent/well after corrected by DNA concentration.

*2. Detection of Hexokinase and Glycogen Synthase Kinase-3 ("GSK-3") proteins:* Western blot analysis was performed by the method of Burnett. Burnett, W.N. Western Blotting: electrophoretic transfer of proteins from sodium dodecyl sulfate--polyacrylamide gels to unmodified nitrocellulose and radiographic detection with antibody and radioiodinated protein A. Anal. Biochem. 112: 195-203 (1981). After washing the skeletal muscle cells with PBS three times, the cells were centrifuged at 600 xg for 5 min. 50 µl of buffer B (100mM Tris/HCl pH 7.4 containing 100 mM sodium pyrophosphate, 100 mM sodium fluoride, 5 mM EDTA, 2 mM sodium orthovanadate, 1 mM PMSF, 20 ug/ml aprotinin and 1% triton x-100) were added to microcentrifuge tubes and sonicated for 20 sec as described above. Sonicates were kept on ice for 30 min. and centrifuged 15000 xg for 10 min. Protein concentration of supernatant were measured. Aliquots of supernatants that normalize to amount of protein were diluted 1:1 in 2x Lamelli's buffer and heated for 5 min at 95°C. Proteins were separated on 8% SDS. PAGE gels and then transferred to nitrocellulose. HK protein was measured by incubating nitrocellulose sheets with anti-HK polyclonal antibody (raised from sheep at 1:250 dilution in TBS buffer containing 3 % BSA) overnight at 4°C, then 15 min x 4 TBS washes, followed by incubation with anti-sheep antibody conjugated with horseradish peroxidase (HRP, 1:75000 dilution). GSK-3 proteins were identified using a monoclonal IgG (0.5 ug/ml) raised against a synthetic peptide (CKQLLHGEPNVSYICSRY), which recognizes GSK-3 at 46-51 kDa (Upstate, Lake Placid, NY). The second antibody was anti-mouse IgG conjugated with HRP 1:5000 dilution (Sigma, St. Louis, MO). After extensive washing with TBS, immune complexes were detected using an enhanced chemiluminescence. kit. The bands on the films were quantified by Alpha Imager 2000 (San Leandro, CA).

*RNA Preparation:* Total RNA from the skeletal muscle biopsy was isolated using guanium thicyanate, phenol-chloroform extraction, and alcohol precipitation. RNA sample was quantified by spectrophotometer. The absorption ratio (260:280 nm) was between 1.8 to 2.0 for all preparations and the integrity of the RNA was verified on agarose gel colored with ethidium bromide. For RT-PCR, fresh isolated total RNA was used.

*GS mRNA Level Analysis:* GS mRNA levels were analyzed using one-step RT-PCR kits (Clontech Laboratories, Inc., Palo Alto, CA). GS first-strand CDNA synthesis was performed from 1 µh of total RNA with 1 X RT-AdvanTaq plus enzyme mix in 40 mM Tricine, 20 mM Kcl, 3 mM MgCl2, 3.75 ug/ml BSA, 0.2mM deoxynucleoside triphosphates, 400 pmol of oligo(dt) primer, and 200 pm GS primers (Sense 5'-GTGCTGACGTCTTTCTGGAG-3', antisense 5'-CCAGCATCTTGTTCATGTCG-3') in a final volume of 50 µl. The reaction mixtures were subjected to incubations for 60 min. at 50°C in the Eppendorf Master gradient cycler (Westbury, NY). The reaction was stopped by heating at 95°C for 5 min. Then PCR mixtures were subjected to 15 cycles of PCR amplification with a cycle profile including denaturation for 30 sec at 95°C, annealing for 30 sec at 65°C, and elongation for 2 min at 72°C, followed by 20 cycles of PCR amplification where a cycle was 1 min at 95°C, 1 min at 55°c, and 2 min at 72°C, respectively. Final extension was 5 min at 72°C. Human G3PDH primers were added to PCR reaction tubes at the same time as the internal control.

*Analysis of RT-PCR Products:* The amplification products of 10 µl of each PCR was separated in a 2% agarose gel, stained with 0.5 µg/ml ethidium bromide, and photographed. The band densities were evaluated (Alpha Imager 2000, San Leandro, CA). The absence of genomic DNA contamination was verified by control experiments that omit RNA or without reverse transcriptase in the RT step by preheating a master mix at 95°C for 5 min to inactivate the enzyme.

*Statistical Analysis:* Statistical significance was evaluated using ANOVA. Comparison was made for insulin sensitivity and gene expression between CrPic and biotin alone and in combination when compared to control. Significance was accepted at the p < 0.05 level.

### Results

Our data strongly suggests that chromium may improve insulin signaling and we have demonstrated this in an animal model representative of the insulin resistance syndrome *in vivo.* Specifically, CrPic versus control solutions were given in hyperinsulinemic, insulin-resistant, obese JCR rats. The study was initiated on animals at approximately 4 months of age, and animals were treated specifically for three months. As demonstrated in Figure 7, CrPic appeared to significantly lower fasting plasma insulin when assessed at the end of study. In addition, when comparing the glucose response to an intraperitoneal glucose tolerance test, CrPic significantly improved glucose response (see Figure 8A), and significantly lowered insulin response in the obese rat (Figure 8B). Insulin sensitivity, as assessed with an insulin tolerance test, was significantly improved with CrPic (Figure 9). This was accomplished without any change in body weight in the animals. The improvement in insulin sensitivity was associated with a reduction in total cholesterol levels compared to lean animals (See, e.g., Table 2, and Figures 10 & 11). The effect of the combination of chromium and biotin on lipid levels in the animal study is detailed in Table 2. Specifically, the effect of chromium and biotin on cholesterol levels, high density lipid cholesterol (HDL-ch) levels, and triglyceride levels are recorded in Table 2.

**Table 2: Chromium Picolinate + Biotin Animal Study (Effect of Combination on Lipid Levels)**

| **Cholesterol** | | | | |
|---|---|---|---|---|
| Month | 0 | 1 | 2 | 3 |
| Obese Control | 99 | 112 | 125 | 144 |
| Low Chromium | 109 | 93 | 130 | 137 |
| Low Biotin | 111 | 117 | 122 | 118 |
| Low Chromium/Low Biotin | 102 | 121 | 128 | 123 |
| Low Biotin/High Chromium | 104 | 102 | 109 | 114 |
| High Chromium | 110 | 112 | 120 | 122 |
| High Biotin | 106 | 111 | 130 | 144 |
| High Biotin/Low Chromium | 107 | 123 | 121 | 124 |
| High Biotin/High Chromium | 104 | 112 | 128 | 121 |
| Lean Control | 49 | 62 | 61 | 64 |

| **HDL-ch** | | | | |
|---|---|---|---|---|
| Month | 0 | 1 | 2 | 3 |
| Obese Control | 44.5 | 40.6 | 38.1 | 38.6 |
| Low Chromium | 45.9 | 38.6 | 43.4 | 45.9 |
| Low Biotin | 47.8 | 46.5 | 45.8 | 47.3 |
| Low Chromium/Low Biotin | 43 | 39.1 | 44.3 | 51.2 |
| Low Biotin/High Chromium | 44.9 | 43.7 | 48.9 | 51.3 |
| High Chromium | 43.1 | 43.2 | 51.5 | 48.1 |
| High Biotin | 43.6 | 42 | 46.8 | 46.9 |
| High Biotin/Low Chromium | 46.5 | 46 | 56 | 56.6 |
| High Biotin/High Chromium | 46.7 | 52.7 | 57.1 | 57.6 |
| Lean Control | 28.4 | 28.1 | 27.2 | 28.1 |

| **Triglyceride mg/dl** | | | | |
|---|---|---|---|---|
| Month | 0 | 1 | 2 | 3 |
| Obese Control | 318 | 293 | 356 | 362 |
| Low Chromium | 319 | 267 | 256 | 242 |
| Low Biotin | 317 | 331 | 272 | 229 |
| Low Chromium/Low Biotin | 286 | 296 | 207 | 206 |
| Low Biotin/High Chromium | 300 | 271 | 209 | 185 |
| High Chromium | 302 | 283 | 221 | 174 |
| High Biotin | 289 | 292 | 294 | 301 |
| High Biotin/Low Chromium | 298 | 336 | 321 | 352 |
| High Biotin/High Chromium | 296 | 254 | 240 | 161 |
| Lean Control | 73 | 73 | 73 | 68 |

Further, the improvement in insulin sensitivity was associated with an improved HDL-C level (Figure 12A) and a lowered cholesterol-HDL ratio (Figure 12B). Finally, the co-administration of chromium and biotin resulted in the lowering of overall triglyceride portfolios in treated animals (Figures 16 and 17).

In summary, these animal studies strongly suggests that chromium and biotin have an *in vivo* effect to improve insulin resistance and components of the insulin resistance syndrome.

### EXAMPLE 4

### Improved Blood Cholesterol Levels via Co-Administration of Chromium and Biotin

An individual presenting with high cholesterol is identified. Chromic tripicolinate and biotin are administered orally at a dose of 500 µg and 5 mg per day, respectively. An increase in HDL cholesterol and reduction of LDL cholesterol in the blood are observed over time.

### Reference EXAMPLE 5

### Attenuation in the Elevation in Blood Glucose Levels in People with Type 2 Diabetes via the Co-Administration of Chromium and Biotin

The effect of chromium and biotin on glycosylated hemoglobin levels in Type 2 diabetics was investigated. Specifically, a determination of the effects of chromium picolinate plus biotin incorporated into a carbohydrate containing beverage on blood glucose control in subjects with type 2 diabetes was initiated. Additionally, the effect of chromium picolinate and biotin on fasting blood glucose levels in type 2 diabetics ingesting a carbohydrate containing beverage was evaluated.

### Study Design

The 12 week, randomized, double-blind, controlled clinical trial was conducted to determine the effects of chromium picolinate with biotin on blood glucose control in people with type 2 diabetes.

Thirty-four subjects were included in the evaluation (24 males, 10 females). Selection of the subjects was based on a number of criteria. Each subject presented with type 2 diabetes, had a fasting glucose level of greater than 90 but less than 170 mg/dL, and a glycosylated hemoglobin level greater than 7 mg/dL. A subject's hemoglobin level must have exceeded 12.5 g/dL and his or her BMI was greater than 26.0 but less than 39.0 kg/m². Baseline hypoglycemic medications remained stable throughout the treatment period.

The study included two parallel groups, a control group and a test group. Each group received beverages containing 29 g of carbohydrate, twice daily as a dietary supplement. The test and control beverages were similar in content to popular beverages designed for people with diabetes and included the following ingredients: water, maltodextrin, soy protein isolate, canola oil, inulin, cocoa powder, defatted, maltodextrin, crystalline fructose, vitamin and mineral mix, natural choclate flavor, lecithin, natural vanilla flavor, Nutrasweet@ (NutraSweet Co., Deerfield, IL), acesulfame K, and SeaKem (FMC Corp., Philadelphia, PA). The test group received beverages containing 300 µg chromium (as chromium picolinate) and 150 µg biotin. Table 3 details the nutritional breakdown of the beverage.

**Table 3: Nutrient Beverage Composition**

| **Nutrients** | **With CrPic + Biotin** | **Control** |
|---|---|---|
| Total Calories, KCal | 200 | 200 |
| Fat, g | 6.8 | 6.8 |
| Protein | 10.0 | 10.0 |
| Carbohydrates, g | 29.0 | 29.0 |
| Dietary Fiber, g | 5.4 | 5.4 |
| Chromium (as CrPic), µg | 300.0 | - |
| Biotin, µg | 150.0 | - |

| | | |
|---|---|---|
| *N.B.* Other vitamins and minerals were the same in both groups (based on % daily value and RDA); saturated fatty acids (SFA), monounsaturated fatty acids (MUFA), and polyunsaturated fatty acids (PUFA) are the same in each test group (source: Canola oil provides high MUFA and PUFA and low SFA). | | |

The duration of the clinical trial was 12 weeks. At the start of the 12 week treatment, baseline data was gathered relating to glycosylated hemoglobin levels, fasting blood glucose concentrations, and fatigue. Indicators of blood glucose control were assessed at baseline, during the study, and at the end of the study. Specifically, subjects were evaluated at 0, 1, 2, 4, 6, 8, and 12 weeks.

### Results

The effects of the co-administration of chromium and biotin on the attenuation in the elevation in blood glucose levels in people with type 2 diabetes who ingested a carbohydrate-containing beverage twice daily were studied. The results of the study are represented in Figures 18, 19, and 20. In Figure 18, the change in mean glycosylated hemoglobin levels from baseline to the end of the week 12 is illustrated. A significant rise in glycosylated hemoglobin levels was observed in the control group given the carbohydrate containing beverage as compared to the treatment group administered chromium picolinate.

In Figure 19, the change in mean fasting blood sugar levels (mg/dL) over the treatment period between subjects given chromium picolinate and biotin versus the control subjects is illustrated. Over time, the control group demonstrated an increase in mean fasting blood sugar levels over time as they continued to consume the carbohydrate-containing beverage. By contrast, subjects who consumed the carbohydrate-containing beverage supplemented with chromium picolinate and biotin did not exhibit a rise in mean fasting blood sugar levels.

Changes in the subjects' energy levels were evaluated at the beginning of the study and after the end of the 12 week trial. Figure 20 is a bar graph depicting differences in fatigue levels in subjects consuming a carbohydrate-containing beverage with chromium picolinate and biotin supplementation versus the control subjects, who consumed the carbohydrate-containing beverage without chromium picolinate and biotin. Fatigue was assessed using a 10-point Likert scale, wherein a score of 0 equaled no fatigue and a score of 10 equaled severe fatigue. Notably, no increase in fatigue was observed in the subjects treated with chromium picolinate and biotin.

### Conclusions

Type 2 diabetic subjects administered a carbohydrate-containing beverage supplemented with chromium picolinate and biotin twice daily for twelve weeks demonstrated a reduced glycosylated hemoglobin level and lower blood glucose level than type 2 diabetic subjects who consumed the carbohydrate-containing beverage without the chromium picolinate and biotin. Without being limited to a particular theory, these phenomena may be based, in part, on the observations that chromium picolinate enhances insulin sensitivity by increasing the number of insulin receptors and/or by facilitating insulin binding at these receptors. Moreover, chromium potentiates the uptake of glucose in muscle cells and increases glycogen production. Biotin stimulates the activity of glucokinase in the liver, improves pancreatic islet cell function, and enhances insulin regulation of chromium III.

Our data has strongly suggested that chromium and biotin may improve post prandial hyperglycemia. As demonstrated in Figures 18 and 19, CrPic and biotin appeared to significantly lower blood glucose and glycated hemoglobin levels when assessed at the end of the study. This was accomplished without any change in fatigue of individuals with chromium picolinate and biotin beverage. In summary, these human studies indicate that CrPic and biotin may effect post prandial hyperglycemia. Moreover, the studies strongly suggest that the co-administration of a chromium complex in concert with biotin may reduce the glycemic index of food.

### Reference EXAMPLE 6

### The co-administration of a chromium complex for the reduction in the glycemic index of a fruit juice

A chromium complex plus biotin act to lower the glycemic index of orange juice. 600 µg of chromium histidinate and 300 µg of biotin are formulated as a liquid and added to eight ounces of orange juice. The chromium histidinate and biotin are mixed with the orange juice and consumed by an individual. A 10-25 mg/dl reduction of blood sugar is observed as compared to the blood sugar of an individual consuming orange juice which has not be supplemented with chromium histidinate and biotin. The glycemic index of the orange juice is lowered.

### Reference EXAMPLE 7

### The co-administration of a chromium complex for the reduction in the glycemic index of a fructose-sweetened carbonated cola-flavored beverage

A chromium complex plus biotin act to lower the glycemic index of a fructose-sweetened carbonated cola-flavored beverage. 600 µg of chromium picolinate and 300 µg of biotin are formulated as a liquid and added to eight ounces of the beverage. The chromium picolinate and biotin are mixed with the beverage and consumed by an individual. A 10-25 mg/dL reduction of blood sugar is observed as compared to the blood sugar of an individual consuming fructose-sweetened carbonated cola-flavored beverage which has not be supplemented with chromium picolinate and biotin. The glycemic index of the fructose-sweetened carbonated cola-flavored beverage is lowered.

### Reference EXAMPLE 8

### Chromium polynicotinate and biotin for the reduction in the glycemic index of a high-carbohyrdrate food

A compound comprising 300 µg chromium polynicotinate and 200 µg biotin is formulated as a powder and sprinkled on top of prepared pasta. The pasta is consumed by an individual. Shortly after the pasta has been consumed, a sample of the individual's blood is tested for glycosylated hemoglobin and blood sugar levels. A reduction in the individual's glycosylated hemoglobin and blood sugar levels is observed. The co. administration of chromium polynicotinate and biotin acts to lower the glycemic index of the pasta, thereby minimizing the sharp elevation in glucose response one would expect to observe when an individual consumes pasta without the chromium polynicotinate and biotin supplementation.

## Claims

1. Use of a chromium complex and biotin in the manufacture of a medicament for the treatment of dyslipidemia.

2. Use of a chromium complex in the manufacture of a medicament for use in combination with biotin for the treatment of dyslipidemia.

3. Use of biotin in the manufacture of a medicament for use in combination with a chromium complex for the treatment of dyslipidemia.

4. The use of any of claims 1 to 3, wherein the effective dose of chromium complex is between 25 and 2,000 micrograms per day.

5. The use of Claim 4, wherein the effective dose of chromium complex is between 300 and 1,000 micrograms per day.

6. The use of any of claims 1 to 3, wherein the effective dose of biotin is between 25 µg and 20 mg per day.

7. The use of Claim 6, wherein the effective dose of biotin is between 150 µg and 5 mg.

8. The use of any preceding claim, wherein said dyslipidemia is caused by elevated levels ofLDL cholesterol in the blood.

9. The use of any of claims 1 to 7, wherein said dyslipidemia is caused by low levels of HDL cholesterol in the blood.

10. The use of any of claims 1 to 7, wherein said dyslipidemia is caused by elevated levels of triglyceride in the blood.

11. The use of any preceding claim, wherein said chromium complex is selected from chromium picolinate, chromic tripicolinate, chromium nicotinate, chromic polynicotinate, chromium chloride, chromium histidinate, and chromium yeasts.

12. The use of any preceding claim, wherein said chromium complex is in a pharmaceutically acceptable carrier.

13. The use of any preceding claim, wherein said biotin is in a pharmaceutically acceptable carrier.

14. The use of any preceding claim, wherein said chromium complex is orally administered.

15. The use of any preceding claim, wherein said biotin is orally administered.

16. The use of any of claims 1 to 13, wherein said chromium complex is for parenteral administration.

17. The use of any of claims 1 to 13 and claim 16, wherein said biotin is parenterally administered.

18. The use of any preceding claim, wherein the medicament further comprises picolinic acid.

19. The use of any preceding claim, wherein the medicament further comprises nicotinic acid.

20. The use of any preceding claim, wherein said chromium complex and said biotin are to be administered simultaneously.

21. The use of any of claims 1 to 19, wherein said biotin is to be administered within 24 hours of said chromium complex.

22. The use of any of claims 1 to 19, wherein said chromium complex is to be administered within 24 hours of said biotin.

## Patentansprüche

1. Verwendung eines Chromkomplexes und von Biotin bei der Herstellung eines Medikaments zur Behandlung von Dyslipidämie.

2. Verwendung eines Chromkomplexes bei der Herstellung eines Medikaments zur Verwendung in Kombination mit Biotin für die Behandlung von Dyslipidämie.

3. Verwendung von Biotin bei der Herstellung eines Medikaments zur Verwendung in Kombination mit einem Chromkomplex für die Behandlung von Dyslipidämie.

4. Verwendung nach einem der Ansprüche 1 bis 3, wobei die wirksame Dosis des Chromkomplexes zwischen 25 und 2.000 Mikrogramm pro Tag beträgt.

5. Verwendung nach Anspruch 4, wobei die wirksame Dosis des Chromkomplexes zwischen 300 und 1.000 Mikrogramm pro Tag beträgt.

6. Verwendung nach einem der Ansprüche 1 bis 3, wobei die wirksame Dosis von Biotin zwischen 25 µg und 20 mg pro Tag beträgt.

7. Verwendung nach Anspruch 6, wobei die wirksame Dosis von Biotin zwischen 150 µg und 5 mg beträgt.

8. Verwendung nach einem der vorangegangenen Ansprüche, wobei die Dyslipidämie durch erhöhte Mengen an LDL-Cholesterol im Blut verursacht wird.

9. Verwendung nach einem der Ansprüche 1 bis 7, wobei die Dyslipidämie durch geringe Mengen an HDL-Cholesterol im Blut verursacht wird.

10. Verwendung nach einem der Ansprüche 1 bis 7, wobei die Dyslipidämie durch erhöhte Mengen an Triglycerid im Blut verursacht wird.

11. Verwendung nach einem der vorangegangenen Ansprüche, wobei der Chromkomplex unter Chrompicolinat, Chromtripicolinat, Chromnicotinat, Chrompolynicotinat, Chromchlorid, Chromhistidinat und Chromhefen ausgewählt ist.

12. Verwendung nach einem der vorangegangenen Ansprüche, wobei der Chromkomplex in einem pharmazeutisch verträglichen Träger vorliegt.

13. Verwendung nach einem der vorangegangenen Ansprüche, wobei das Biotin in einem pharmazeutisch verträglichen Träger vorliegt.

14. Verwendung nach einem der vorangegangenen Ansprüche, wobei der Chromkomplex oral verabreicht wird.

15. Verwendung nach einem der vorangegangenen Ansprüche, wobei das Biotin oral verabreicht wird.

16. Verwendung nach einem der Ansprüche 1 bis 13, wobei der Chromkomplex für die parenterale Verabreichung vorgesehen ist.

17. Verwendung nach einem der Ansprüche 1 bis 13 und Anspruch 16, wobei das Biotin parenteral verabreicht wird.

18. Verwendung nach einem der vorangegangenen Ansprüche, wobei das Medikament weiterhin Picolinsäure umfaßt.

19. Verwendung nach einem der vorangegangenen Ansprüche, wobei das Medikament weiterhin Nicotinsäure umfaßt.

20. Verwendung nach einem der vorangegangenen Ansprüche, wobei der Chromkomplex und das Biotin gleichzeitig zu verabreichen sind.

21. Verwendung nach einem der Ansprüche 1 bis 19, wobei das Biotin innerhalb von 24 Stunden nach dem Chromkomplex zu verabreichen ist.

22. Verwendung nach einem der Ansprüche 1 bis 19, wobei der Chromkomplex innerhalb von 24 Stunden nach dem Biotin zu verabreichen ist.

## Revendications

1. Utilisation d'un complexe de chrome et de biotine dans la production d'un médicament destiné au traitement de la dyslipidémie.

2. Utilisation d'un complexe de chrome dans la production d'un médicament destiné à être utilisé en association avec la biotine pour le traitement de la dyslipidémie.

3. Utilisation de biotine dans la production d'un médicament destiné à être utilisé en association avec un complexe de chrome pour le traitement de la dyslipidémie.

4. Utilisation suivant l'une quelconque des revendications 1 à 3, dans laquelle la dose efficace de complexe de chrome est comprise entre 25 et 2000 microgrammes par jour.

5. Utilisation suivant la revendication 4, dans laquelle la dose efficace de complexe de chrome est comprise entre 300 et 1000 microgrammes par jour.

6. Utilisation suivant l'une quelconque des revendications 1 à 3, dans laquelle la dose efficace de biotine est comprise entre 25 µg et 20 mg par jour.

7. Utilisation suivant la revendication 6, dans laquelle la dose efficace de biotine est comprise entre 150 µg et 5 mg.

8. Utilisation suivant l'une quelconque des revendications précédentes, dans laquelle ladite dyslipidémie est provoquée par des taux élevés de cholestérol-LDL dans le sang.

9. Utilisation suivant l'une quelconque des revendications 1 à 7, dans laquelle ladite dyslipidémie est provoquée par des taux bas de cholestérol-HDL dans le sang.

10. Utilisation suivant l'une quelconque des revendications 1 à 7, dans laquelle ladite dyslipidémie est provoquée par des taux élevés de triglycérides dans le sang.

11. Utilisation suivant l'une quelconque des revendications précédentes, dans laquelle ledit complexe de chrome est choisi entre le picolinate de chrome, le tripicolinate de chrome, le nicotinate de chrome, le polynicotinate de chrome, le chlorure de chrome, l'histidinate de chrome et des levures au chrome.

12. Utilisation suivant l'une quelconque des revendications précédentes, dans laquelle ledit complexe de chrome est présent dans un support pharmaceutiquement acceptable.

13. Utilisation suivant l'une quelconque des revendications précédentes, dans laquelle ladite biotine est présente dans un support pharmaceutiquement acceptable.

14. Utilisation suivant l'une quelconque des revendications précédentes, dans laquelle ledit complexe de chrome est administré par voie orale.

15. Utilisation suivant l'une quelconque des revendications précédentes, dans laquelle ladite biotine est administrée par voie orale.

16. Utilisation suivant l'une quelconque des revendications 1 à 13, dans laquelle ledit complexe de chrome est destiné à l'administration parentérale.

17. Utilisation suivant l'une quelconque des revendications 1 à 13 et la revendication 16, dans laquelle ladite biotine est administrée par voie parentérale.

18. Utilisation suivant l'une quelconque des revendications précédentes, dans laquelle le médicament comprend en outre de l'acide picolinique.

19. Utilisation suivant l'une quelconque des revendications précédentes, dans laquelle le médicament comprend en outre de l'acide nicotinique.

20. Utilisation suivant l'une quelconque des revendications précédentes, dans laquelle ledit complexe de chrome et ladite biotine sont destinés à être administrés simultanément.

21. Utilisation suivant l'une quelconque des revendications 1 à 19, dans laquelle ladite biotine est destinée à être administrée dans les 24 heures de l'administration dudit complexe de chrome.

22. Utilisation suivant l'une quelconque des revendications 1 à 19, dans laquelle ledit complexe de chrome est destiné à être administré dans les 24 heures de l'administration de ladite biotine.
